# EUROPÄISCHE PATENTANMELDUNG

(11) **EP 3 868 420 A1**
(43) Veröffentlichungstag der Anmeldung: **25.08.2021**
(21) Anmeldenummer: 20158251.7
(22) Anmeldetag: 19.02.2020
(51) Int. Cl.: A61M 5/315, B01L 3/00, A61M 5/178, B65B 3/00, A61M 5/00, A61J 1/20, A61M 5/168

(54) **DOSIERBEHÄLTER ZUM DOSIEREN VISKOSER MATERIALKOMPONENTEN**

(71) Anmelder: Ivoclar Vivadent AG, 9494 Schaan (LI)
(72) Erfinder: Senn, Bruno, 9056 Gais (CH)
(74) Vertreter: Baldus, Oliver

(57) **Zusammenfassung**

Die vorliegende Erfindung betrifft einen Dosierbehälter (100-1, 100-2) zum Dosieren einer viskosen Materialkomponente (103-1, 103-2), mit einem Kolben (105-1, 105-2) zum Drücken der Materialkomponente (103-1, 103-2) aus einem Vorratsbehälter (111-1, 111-2) in eine Dosierkammer (107 1, 107-2); und einem Dosierstößel (109-1, 109-2) zum Anzeigen einer Menge der Materialkomponente (103-1, 103-2) in der Dosierkammer (107-1, 107-2), über den die Materialkomponente (103-1, 103-2) aus der Dosierkammer (107-1, 107-2) ausgebbar ist.

## Beschreibung

Die vorliegende Erfindung betrifft einen Dosierbehälter zum Dosieren einer viskosen Materialkomponente, ein Dosiersystem mit einem ersten und einem zweiten Dosierbehälter und ein Verfahren zum Dosieren von viskosen Materialkomponenten.

Beim Dosieren von viskosen Materialkomponenten kann es zu Ungenauigkeiten kommen. Das manuelle Dosieren von viskosen Materialkomponenten ist zeitintensiv und ungenau.

Es ist daher die Aufgabe der vorliegenden Erfindung, das Dosieren viskoser Materialkomponenten zu vereinfachen und zu beschleunigen.

Diese technische Aufgabe wird durch Gegenstände mit den Merkmalen nach den unabhängigen Ansprüchen gelöst. Vorteilhafte Ausführungsformen sind Gegenstand der Unteransprüche, der Beschreibung und der Zeichnungen.

Gemäß einem ersten Aspekt wird die technische Aufgabe durch einen Dosierbehälter zum Dosieren einer viskosen Materialkomponente gelöst, mit einem Kolben zum Drücken der Materialkomponente aus einem Vorratsbehälter in eine Dosierkammer; und einem Dosierstößel zum Anzeigen einer Menge der Materialkomponente in der Dosierkammer, über den die Materialkomponente aus der Dosierkammer ausgebbar ist. Durch den Dosierbehälter wird der technische Vorteil erreicht, dass die auszugebende Menge der Materialkomponente über den Dosierstößel genau bestimmt werden kann.

In einer technisch vorteilhaften Ausführungsform des Dosierbehälters sind der Vorratsbehälter und/oder die Dosierkammer zylinderförmig. Dadurch wird beispielsweise der technische Vorteil erreicht, dass sich der Kolben und/oder der Dosierstößel auf einfache Weise herstellen lassen.

In einer weiteren technisch vorteilhaften Ausführungsform des Dosierbehälters umfasst der Dosierbehälter eine Zwischensperrvorrichtung zum Sperren eines Verbindungskanals zwischen dem Vorratsbehälter und der Dosierkammer. Dadurch wird beispielsweise der technische Vorteil erreicht, dass ein unbeabsichtigtes Mischen unterschiedlicher Materialkomponenten verhindert werden kann.

In einer weiteren technisch vorteilhaften Ausführungsform des Dosierbehälters umfasst der Dosierbehälter eine Ausgabesperrvorrichtung zum Sperren einer Ausgabeöffnung für die Materialkomponente. Dadurch wird beispielsweise der technische Vorteil erreicht, dass ein unbeabsichtigtes Ausgeben der Materialkomponente verhindert werden kann.

In einer weiteren technisch vorteilhaften Ausführungsform des Dosierbehälters umfasst der Dosierbehälter eine Dosierkammer für eine weitere Materialkomponente. Dadurch wird beispielsweise der technische Vorteil erreicht, dass zwei Materialkomponenten durch ein und denselben Dosierbehälter ausgegeben werden können.

Gemäß einem ersten Aspekt wird die technische Aufgabe durch ein Dosiersystem mit einem ersten Dosierbehälter nach dem ersten Aspekt und einem zweiten Dosierbehälter gelöst, wobei der Dosierstößel für die Materialkomponente des ersten Dosierbehälters und der Dosierstößel für die Materialkomponente des zweiten Dosierbehälters mechanisch miteinander verbunden sind. Durch das Dosiersystem wird beispielsweise der technische Vorteil erreicht, dass die beiden Materialkomponenten gleichzeitig mit einer Bewegung ausgegeben werden können.

In einer technisch vorteilhaften Ausführungsform des Dosiersystems sind der Kolben des ersten Dosierbehälters und der Kolben des zweiten Dosierbehälters parallel zueinander angeordnet. Dadurch wird beispielsweise der technische Vorteil erreicht, dass beide Kolben gleichzeitig durch die gleiche Bewegung betätigt werden können.

In einer weiteren technisch vorteilhaften Ausführungsform des Dosiersystems umfasst das Dosiersystem eine Dosierspitze mit einem ersten Kanal für die Materialkomponente des ersten Dosierbehälters und einem zweiten Kanal für die Materialkomponente des zweiten Dosierbehälters. Dadurch wird beispielsweise der technische Vorteil erreicht, dass sich die beiden Materialkomponente getrennt durch das Dosiersystem ausgeben lassen.

In einer weiteren technisch vorteilhaften Ausführungsform des Dosiersystems bildet die Dosierspitze eine Zwischensperrvorrichtung und/oder Ausgabesperrvorrichtung des ersten Dosierbehälters und des zweiten Dosierbehälters. Die Dosierspitze kann beispielsweise zwischen einer ersten Position zum Sperren des Verbindungskanals und einer zweiten Position zum Sperren der Ausgabeöffnung beweglich sein. Dadurch wird beispielsweise der technische Vorteil erreicht, dass die Ausgabeöffnung und der Verbindungskanal sich auf einfache Weise fluidtechnisch sperren und entsperren lassen.

In einer weiteren technisch vorteilhaften Ausführungsform des Dosiersystems weist der Dosierstößel für die erste Materialkomponente eine Länge auf, dass dieser bis an das Ende des ersten Kanals reicht und/oder der Dosierstößel für die zweite Materialkomponente weist eine Länge auf, dass dieser bis an das Ende des zweiten Kanals reicht. Dadurch wird beispielsweise der technische Vorteil erreicht, dass die dosierten Materialkomponenten vollständig aus dem Dosiersystem ausgegeben werden können.

In einer weiteren technisch vorteilhaften Ausführungsform des Dosiersystems umfasst das Dosiersystem einen Mischbehälter zum Aufnehmen der ersten und der zweiten Materialkomponente. Dadurch wird beispielsweise der technische Vorteil erreicht, dass sich die beiden Materialkomponenten intern mischen lassen.

In einer weiteren technisch vorteilhaften Ausführungsform des Dosiersystems ist der Mischbehälter auf die Dosierspitze aufsteckbar. Dadurch wird beispielsweise der technische Vorteil erreicht, dass sich die vermischten Materialkomponenten auf einfache Weise aus dem Dosiersystem entnehmen lassen.

In einer weiteren technisch vorteilhaften Ausführungsform des Dosiersystems ist der Mischbehälter auf der Dosierspitze durch Eindringen der ersten und zweiten Materialkomponente verschiebbar. Dadurch wird beispielsweise der technische Vorteil erreicht, dass das Einführen und das Vermischen der Materialkomponenten ohne das Eindringen von Luft durchgeführt werden kann.

In einer weiteren technisch vorteilhaften Ausführungsform des Dosiersystems sind der erste Dosierbehälter und der zweite Dosierbehälter voneinander trennbar. Dadurch wird beispielsweise der technische Vorteil erreicht, dass sich unterschiedliche Materialkomponenten miteinander kombinieren und Mischen lassen.

Gemäß einem dritten Aspekt wird die technische Aufgabe durch ein Verfahren zum Dosieren von viskosen Materialkomponenten gelöst, mit den Schritten eines Drückens der Materialkomponente aus einem Vorratsbehälter in eine Dosierkammer; und eines Ausgebens der Materialkomponente aus der Dosierkammer über einen Dosierstößel. Durch das Verfahren werden die gleichen technische Vorteile wie durch den Dosierbehälter nach dem ersten Aspekt erreicht.

Ausführungsbeispiele der Erfindung sind in den Zeichnungen dargestellt und werden im Folgenden näher beschrieben.

Es zeigen:
- Fig. 1: eine Querschnittsansicht eines Dosiersystems mit einem ersten und einem zweiten Dosierbehälter;
- Fig. 2: eine Querschnittsansicht des Dosiersystems mit einer Dosierspitze;
- Fig. 3: eine Querschnittsansicht des Dosiersystems mit einer Zwischensperrvorrichtung;
- Fig. 4: eine Außenansicht des Dosiersystems mit verschiebbarer Dosierspitze;
- Fig. 5: eine Querschnittsansicht einer Zwischensperrvorrichtung und Ausgabesperrvorrichtung in einer ersten Position;
- Fig. 6: eine Querschnittsansicht der Zwischensperrvorrichtung und Ausgabesperrvorrichtung in einer zweiten Position; und
- Fig. 7: ein Blockdiagramm eines Verfahrens Verfahren zum Dosieren von viskosen Materialkomponenten.

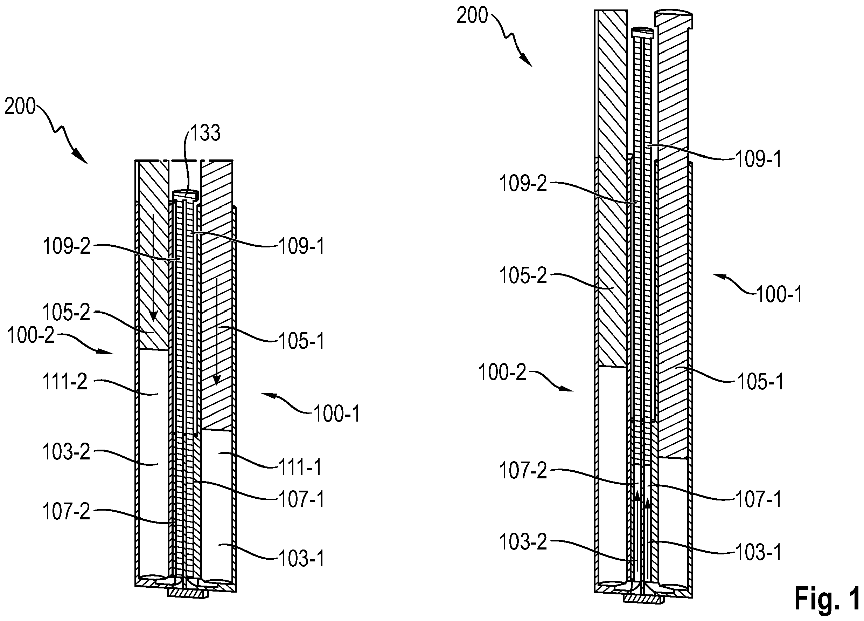

Fig. 1 zeigt eine Querschnittsansicht eines Dosiersystems 200 mit einem ersten und einem zweiten Dosierbehälter 100-1 und 100-2. Der erste Dosierbehälter 100-1 umfasst einen zylindrischen Vorratsbehälter 111-1, in dem sich eine erste viskose Materialkomponente 103-1 befindet. Bei dieser Materialkomponente 103-1 handelt es sich beispielsweise um eine viskose dentale Kunststoffmasse aus viskosem Material für eine Zahnfüllung oder Dentalformulierung, die anschießend gehärtet wird. Die Materialkomponente 103-1 weist eine Viskosität von 3000 - 50.000 Pas auf. Im Allgemeinen können jedoch auch andere viskose Materialkomponente 103-1 verwendet werden, die für das Dosiersystem geeignet sind.

Durch Drücken eines Kolbens 105-1 in Pfeilrichtung wird die viskose Materialkomponente 103-1 aus dem Vorratsbehälter 111-1 in eine zylindrische Dosierkammer 107-1 gedrückt. Bei diesem Vorgang bewegt sich der Dosierstößel 109-1 in eine entgegengesetzte Richtung. Dadurch wird die eingeschlossene Luft in der Dosierkammer 107-1 minimiert. Anhand des Hubes des Dosierstößel 109-1 kann die Menge und das Volumen der Materialkomponente 103-1 bestimmt werden, die sich in der Dosierkammer 107-1 befindet. Da die Dosierkammer 107-1 einen geringeren Durchmesser als der Vorratsbehälter 111-1 aufweist, kann die Menge der Materialkomponente 103-1 in der Dosierkammer 107-1 mit einer hohen Genauigkeit bestimmt werden.

Wird der Dosierstößel 109-1 anschließend in die andere Richtung bewegt, lässt sich die in der Dosierkammer 107-1 befindliche Materialkomponente 103-1 wiederum aus der Dosierkammer 107-1 dem Dosiersystems 200 ausgeben.

Zusätzlich umfasst der Dosierbehälter 100-1 eine zweite Dosierkammer 107-2 für eine weitere Materialkomponente 103-2 aus einem anderen Vorratsbehälter 111-2. Bei dieser Materialkomponente 103-2 handelt es sich beispielsweise um eine viskose dentale Materialkomponente aus viskosem Kunststoff für eine Zahnfüllung oder Dentalformulierung, die zum Färben oder Härten verwendet wird. Die Materialkomponente 103-2 weist eine Viskosität von 3000 - 50.000 Pas auf. Im Allgemeinen können jedoch auch andere viskose Materialkomponente 103-2 verwendet werden, die für das Dosiersystem geeignet sind.

Die Materialkomponenten 103-1 oder 103-2 weisen beispielsweise eine Dichte von ca. 2.1 g/cm³ auf. Die Menge der Materialkomponenten 103-1 oder 103-2 je Vorratsbehälter 111-1 oder 111-2 kann ca. 10 g betragen. Die Dosiermenge je Materialkomponente 103-1 oder 103-2 kann 0.15 g bis 0.5 g betragen. Im Allgemeinen können hier jedoch auch andere Werte gewählt werden.

Dieser Vorratsbehälter 111-2 ist fluidtechnisch derart mit dem Vorratsbehälter 111-1 gekoppelt, dass die Materialkomponente 103-2 aus dem Vorratsbehälter 111-2 in die andere Dosierkammer 107-2 strömen kann. Dies geschieht durch Drücken des zweiten Kolbens 105-2 in Pfeilrichtung, der parallel zu dem ersten Kolben 105-1 angeordnet ist. Dabei bewegt sich der zweite Dosierstößel 109-2 ebenfalls in die entgegensetzte Richtung und zeigt dadurch die Menge der in der Dosierkammer 107-2 befindlichen Materialkomponente 103-2 an. Auch diese Materialkomponente 103-2 kann über den zweiten Dosierstößel 109-2 wieder aus der Dosierkammer 107-2 ausgegeben werden.

Die parallel angeordneten Dosierstößel 109-1 und 109-2 können über einen Steg 133 mechanisch miteinander gekoppelt oder verbunden sein, so dass sich diese zusammen bewegen lassen. Die Dosierbehälter 100-1 und 100-2 und die übrigen Teile sind beispielsweise durch entsprechende Kunststoffformteile gebildet. Durch das Dosiersystem 200 ist die Genauigkeit der Dosierung unabhängig vom Komprimierverhalten der Materialkomponente 103-1 und 103-1. Das Dosiersystem 200 weist ein geringes Totvolumen auf, so das wenig Restmaterial in den Dosierbehältern 100-1 und 100-2 verbleibt.

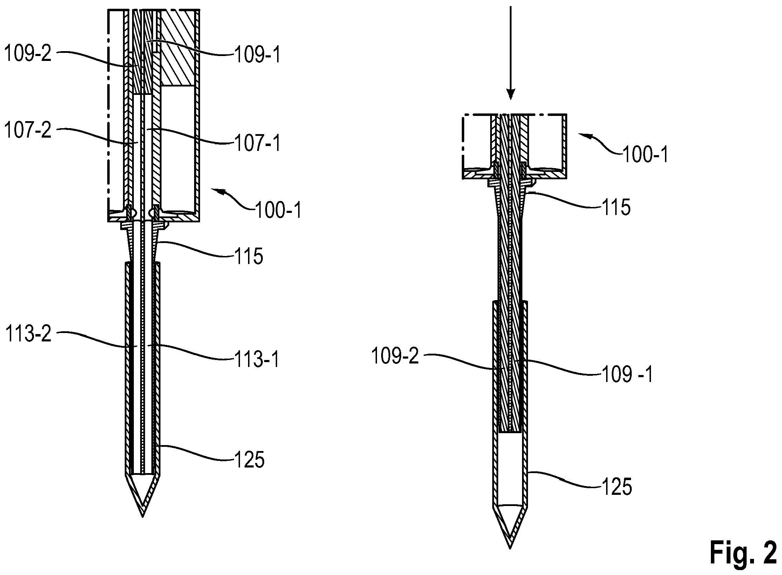

Fig. 2 zeigt eine Querschnittsansicht des Dosiersystems 200 mit einer Dosierspitze 115. Die Dosierspitze 115 ist beispielsweise durch ein Kunststoffteil gebildet, in dem ein erster Kanal 113-1 für die Materialkomponente 103-1 und ein zweiter Kanal 113-2 für die Materialkomponente 103-2 gebildet sind. Der erste und der zweite Kanal 113-1 und 113-2 weisen jeweils den gleichen Durchmesser wie die jeweilige Dosierkammer 107-1 und 107-2 auf.

Die Länge der Dosierstößel 109-1 und 109-2 ist derart gewählt, dass diese die Materialkomponente 103-1 und 103-2 vollständig aus den Kanälen 113-1 und 113-2 drücken können. Auf diese Weise lassen sich die Materialkomponenten 103-1 und 103-2 vollständig aus den Dosierkammern 107-1 und 107-2 und aus dem Dosiersystem 200 entfernen.

Am unteren Ende der Dosierspitze 115 kann ein Mischbehälter 125 aufgesetzt sein, der zum Aufnehmen der ersten und der zweiten Materialkomponente 103-1 und 103-2 dient. Beim Ausgeben der Materialkomponenten 103-1 und 103-2 aus dem Dosiersystem 200 gleitet der verschiebbare Mischbehälter 125 von der Dosierspitze 115. Auf diese Weise können die Materialkomponenten 103-1 und 103-2 in dem Mischbehälter 125 vermischt werden, ohne dass Luft in den Mischbehälter 125 eindringt.

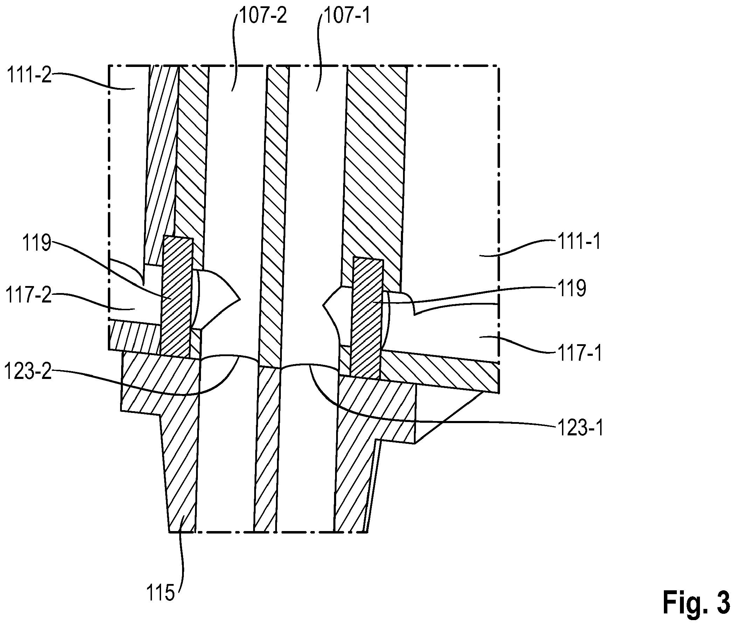

Fig. 3 zeigt eine Querschnittsansicht des Dosiersystems 200 mit einer Zwischensperrvorrichtung 119. Die Zwischensperrvorrichtung 119 dient zum Sperren eines Verbindungskanals 117-1 und 117-2, der zwischen dem Vorratsbehälter 111-1 oder 111-2 und der Dosierkammer 107-1 oder 107-2 verläuft. Durch den Verbindungskanal 117-1 und 117-2 strömt die viskose Materialkomponente 103-1 und 103-2 aus den jeweiligen Vorratsbehältern 111-1 und 111-2 in die Dosierkammern 107-1 und 107-2. Durch die Zwischensperrvorrichtung 119 kann ein Durchfluss der Materialkomponenten 103-1 und 103-2 wahlweise gestoppt werden. Zu diesem Zweck lässt sich die Zwischensperrvorrichtung 119 zwischen unterschiedlichen Positionen bewegen.

Die Zwischensperrvorrichtung 119 kann beispielsweise mechanisch mit der beweglichen Dosierspitze 115 gekoppelt sein. Wird die Dosierspitze 115 in eine bestimmte Position bewegt, können die Verbindungskanäle 117-1 und 117-2 gesperrt werden. Auf diese Weise lässt sich verhindern, dass die Materialkomponenten 103-1 und 103-2 bei Ausdrücken zurück in die Vorratsbehälter 111-1 und 111-2 strömen.

Die Zwischensperrvorrichtung 119 kann beispielsweise durch eine bewegliche Scheibe gebildet sein, die in den jeweiligen Verbindungskanal 117-1 und 117-2 geschoben wird. Die Zwischensperrvorrichtung 119 kann aber auch ein Rückschlagventil umfassen, dass den Rückfluss der Materialkomponenten 103-1 oder 103-2 in die Vorratsbehälter 111-1 und 111-2 verhindert. Die Ausgabeöffnungen 123-1 und 123-2 sind für die Ausgabe der beiden Materialkomponenten 103-1 und 103-2 freigegeben, so dass diese durch die beiden Kanäle 113-1 und 113-2 der Dosierspitze 115 fließen können.

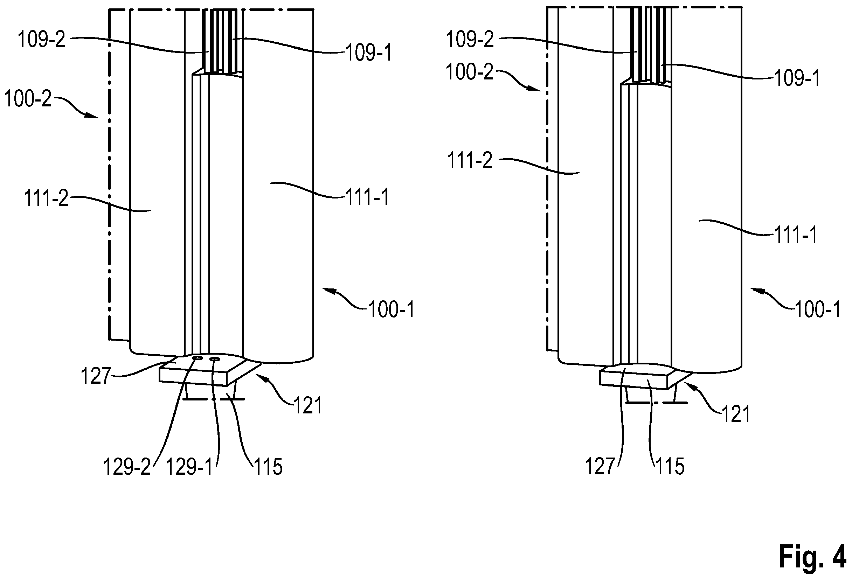

Fig. 4 zeigt eine Außenansicht des Dosiersystems 200 mit verschiebbarer Dosierspitze 115. Die verschiebbare Dosierspitze 115 umfasst eine anliegende Platte 127, in der zwei Durchtrittsöffnungen 129-1 und 129-2 gebildet sind. Dadurch wird eine Ausgabesperrvorrichtung 121 gebildet. In der ersten Position der Platte 127 liegt deren geschlossene Fläche vor den Ausgabeöffnungen 123-1 und 123-2 der Dosierkammern 107-1 oder 107-2, so dass ein Austritt der Materialkomponenten 103-1 und 103-2 aus den Dosierkammern 107-1 oder 107-2 verhindert wird. In dieser Position sind die Verbindungskanäle 117-1 und 117-2 im Inneren des Dosiersystems 200 freigegeben.

Die Dosierspitze 115 kann linear zur Seite bewegt werden. Danach befindet sich die Platte 127 in einer zweiten Position, bei der die jeweiligen Durchtrittsöffnungen 129-1 und 129-2 und die Ausgabeöffnungen 123-1 und 123-2 übereinander liegen, so dass die Materialkomponenten 103-1 und 103-2 aus den Dosierkammern 107-1 oder 107-2 austreten können. Die verschlossenen Ausgabeöffnungen 123-1 und 123-2 der Dosierkammern 107-2 werden dadurch zur Dosierspitze 115 frei. In dieser Position sind die Verbindungskanäle 117-1 und 117-2 im Inneren des Dosiersystems 200 gesperrt.

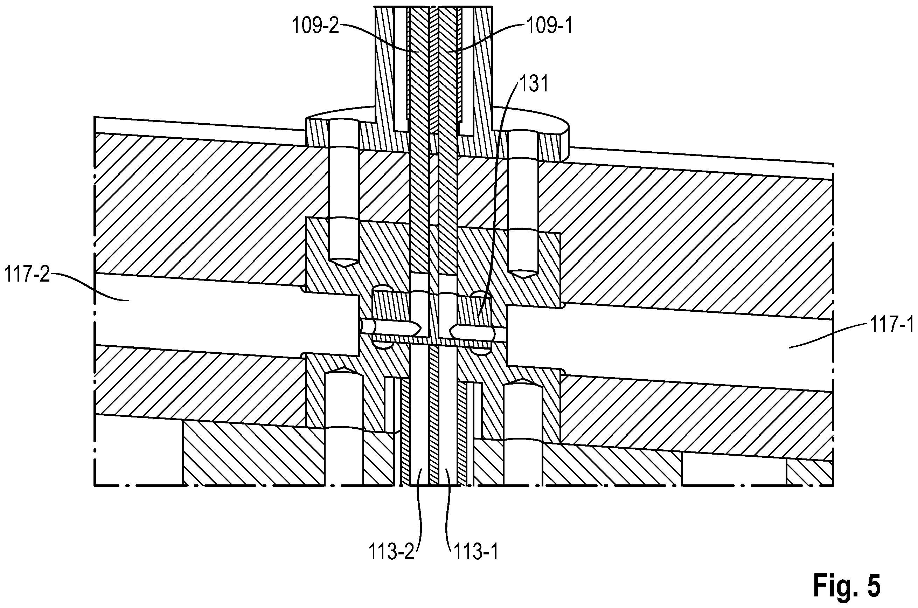

Fig. 5 zeigt eine Querschnittsansicht einer Zwischensperrvorrichtung 119 und Ausgabesperrvorrichtung 121 in einer ersten Position. Hierbei sind sowohl die Zwischensperrvorrichtung 119 als auch die Ausgabesperrvorrichtung 121 durch ein gemeinsames Schiebeteil 131 gebildet. In der gezeigten Position gibt das Schiebeteil 131 die Verbindungskanäle 117-1 und 117-2 im Inneren des Dosiersystems 200 frei, so dass die Materialkomponenten 103-1 und 103-2 durch diese aus den Vorratsbehältern 111-1 und 111-2 in die Dosierkammern 107 1 oder 107-2 gedrückt werden können. Die Ausgabeöffnungen 123-1 und 123-2 sind hingegen durch das Schiebeteil 131 gesperrt, so dass ein Austreten der Materialkomponenten 103-1 und 103-2 verhindert wird.

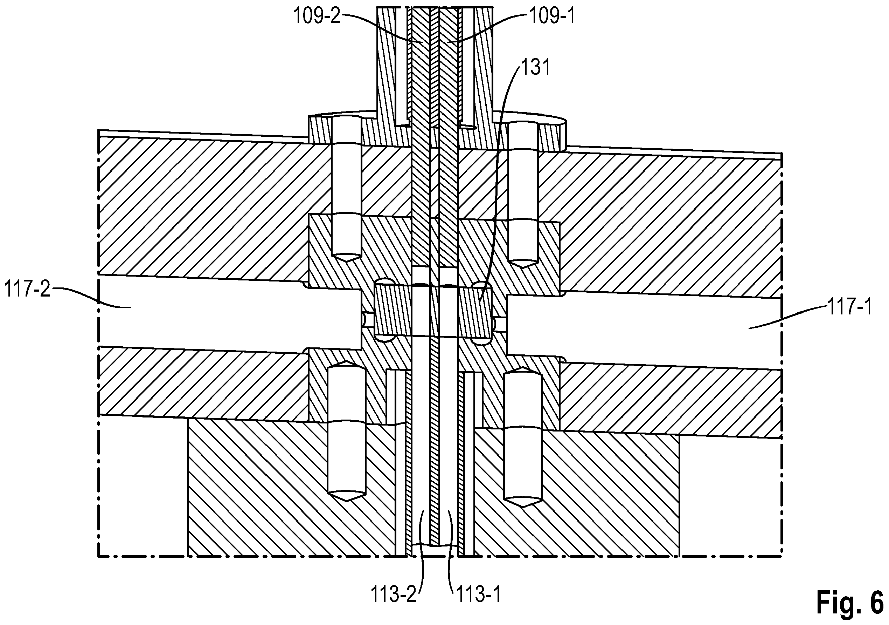

Fig. 6 zeigt eine Querschnittsansicht der Zwischensperrvorrichtung 119 und Ausgabesperrvorrichtung 121 in einer zweiten Position. In dieser Position gibt das Schiebeteil 131 die Ausgabeöffnungen 123-1 und 123-2 frei, so dass die die Materialkomponenten 103-1 und 103-2 aus den Dosierkammern 107-1 oder 107-2 in die Kanäle 113-1 und 113-2 der Dosierspitze 115 austreten können. Die Verbindungskanäle 117-1 und 117-2 im Inneren sind hingegen durch das Schiebeteil 131 gesperrt, so dass ein Rückfluss der Materialkomponenten 103-1 und 103-2 in die Dosierkammern 107-1 oder 107-2 verhindert wird.

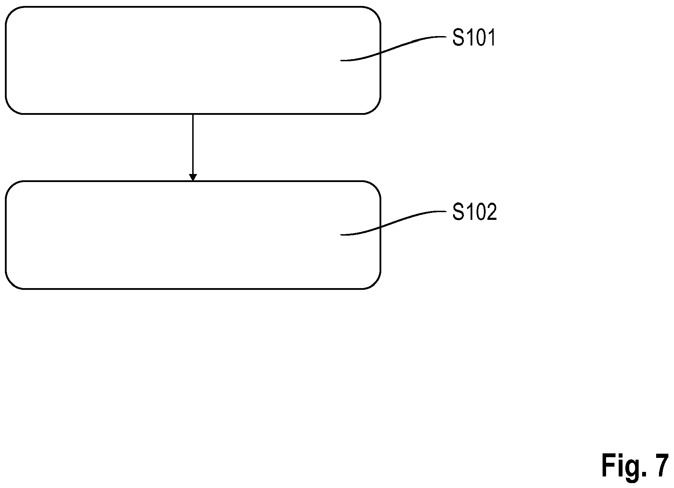

Fig. 7 zeigt ein Blockdiagramm eines Verfahrens zum Dosieren von viskosen Materialkomponenten 103-1 und 103-2. In einem ersten Schritt wird die Materialkomponente 103-1 oder 103-2 aus dem Vorratsbehälter 111-1 oder 111-2 durch die Kolben 105-1 und 105-2 in die Dosierkammer 107-1 oder 107-2 gedrückt. Dabei bewegt sich durch die eindringende Materialkomponente 1053-1 und 103-2 der Dosierstößel 109-1 oder 109-2 aus der Dosierkammer 107-1 oder 107-2 heraus. Danach werden die Zwischensperrvorrichtung 119 und die Ausgabesperrvorrichtung 121 umgeschaltet.

Anschließend wird in Schritt S102 die Materialkomponente 103-1 oder 103-2 wieder aus der Dosierkammer 107-1 oder 107-2 über den Dosierstößel 109-1 oder 109-2 vollständig ausgegeben. Dies geschieht, indem der Dosierstößel 109-1 oder 109-2 in die entgegengesetzte Richtung bewegt wird. Dieses Verfahren kann auch mit gekoppelten Dosierbehältern 100-1 und 100-2 durchgeführt werden. Dabei lassen sich die beiden Dosierstößel 109-1 oder 109-2 gleichzeitig bewegen, so dass beide Materialkomponenten 103-1 oder 103-2 gleichzeitig aus dem Dosiersystem 200 ausgegeben werden.

Durch das Verfahren ist die Genauigkeit der Dosierung unabhängig vom Komprimierverhalten der Materialkomponenten 103-1 und 103-2. Es entsteht ein geringes Totvolumen, so dass wenig Restmaterial in dem Dosiersystem 200 verbleibt.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Dosierbehälter
- 103: Materialkomponente
- 105: Kolben
- 107: Dosierkammer
- 109: Dosierstößel
- 111: Vorratsbehälter
- 113: Kanal
- 115: Dosierspitze
- 117: Verbindungskanal
- 119: Zwischensperrvorrichtung
- 121: Ausgabesperrvorrichtung
- 123: Ausgabeöffnung
- 125: Mischbehälter
- 127: Platte
- 129: Durchtrittsöffnungen
- 131: Schiebeteil
- 133: Steg

## Patentansprüche

1. Dosierbehälter (100-1, 100-2) zum Dosieren einer viskosen Materialkomponente (103-1, 103-2), mit:
einem Kolben (105-1, 105-2) zum Drücken der Materialkomponente (103-1, 103-2) aus einem Vorratsbehälter (111-1, 111-2) in eine Dosierkammer (107-1, 107-2); und
einem Dosierstößel (109-1, 109-2) zum Anzeigen einer Menge der Materialkomponente (103-1, 103-2) in der Dosierkammer (107-1, 107-2), über den die Materialkomponente (103-1, 103-2) aus der Dosierkammer (107-1, 107-2) ausgebbar ist.

2. Dosierbehälter (100-1, 100-2) nach Anspruch 1, wobei der Vorratsbehälter (111-1, 111-2) und/oder die Dosierkammer (107-1) zylinderförmig sind.

3. Dosierbehälter (100-1, 100-2) nach einem der vorangehenden Ansprüche, wobei der Dosierbehälter (100-1, 100-2) eine Zwischensperrvorrichtung (119) zum Sperren eines Verbindungskanals (117-1, 117-2) zwischen dem Vorratsbehälter (111-1, 111-2) und der Dosierkammer (107-1, 107-2) umfasst.

4. Dosierbehälter (100-1, 100-2) nach einem der vorangehenden Ansprüche, wobei der Dosierbehälter (100-1, 100-2) eine Ausgabesperrvorrichtung (121) zum Sperren einer Ausgabeöffnung (123-1, 123-2) für die Materialkomponente (103-1, 103-2) umfasst.

5. Dosierbehälter (100-1, 100-2) nach einem der vorangehenden Ansprüche, wobei der Dosierbehälter (100-1, 100-2) eine Dosierkammer (107-2) für eine weitere Materialkomponente (103-2) umfasst.

6. Dosiersystem (200) mit einem ersten Dosierbehälter (100-1) nach einem der Ansprüche 1 bis 5 und einem zweiten Dosierbehälter (100-2), wobei der Dosierstößel (109-1) für die Materialkomponente (103-1) des ersten Dosierbehälters (100-1) und der Dosierstößel (109-2) für die Materialkomponente (103-2) des zweiten Dosierbehälters (100-2) mechanisch miteinander verbunden sind.

7. Dosiersystem (200) nach Anspruch 6, wobei der Kolben (105-1) des ersten Dosierbehälters (100-1) und der Kolben (105-2) des zweiten Dosierbehälters (100-2) parallel zueinander angeordnet sind.

8. Dosiersystem (200) nach einem der Ansprüche 5 oder 6, wobei das Dosiersystem (200) eine Dosierspitze (115) mit einem ersten Kanal (113-1) für die Materialkomponente (103-1) des ersten Dosierbehälters (100-1) und einem zweiten Kanal (113-2) für die Materialkomponente (103-2) des zweiten Dosierbehälters (100-1) umfasst.

9. Dosiersystem (200) nach Anspruch 8, wobei die Dosierspitze (115) eine Zwischensperrvorrichtung (119) und/oder Ausgabesperrvorrichtung (121) des ersten Dosierbehälters (100-1) und des zweiten Dosierbehälters (100-2) bildet.

10. Dosiersystem (200) nach Anspruch 9, wobei der Dosierstößel (109-1) für die erste Materialkomponente (103-1) eine Länge aufweist, dass dieser bis an das Ende des ersten Kanals (113-1) reicht und/oder der Dosierstößel (109-2) für die zweite Materialkomponente (103-2) eine Länge aufweist, dass dieser bis an das Ende des zweiten Kanals (113-2) reicht.

11. Dosiersystem (200) nach einem der Ansprüche 5 bis 10, wobei das Dosiersystem (200) einen Mischbehälter (125) zum Aufnehmen der ersten und der zweiten Materialkomponente (103-1, 103-2) umfasst.

12. Dosiersystem (200) nach Anspruch 11, wobei der Mischbehälter (125) auf die Dosierspitze (115) aufsteckbar ist.

13. Dosiersystem (200) nach Anspruch 12, wobei der Mischbehälter (125) auf der Dosierspitze (115) durch Eindringen der ersten und zweiten Materialkomponente (103-1, 103-2) verschiebbar ist.

14. Dosiersystem (200) nach einem der Ansprüche 5 bis 13, wobei der erste Dosierbehälter (100-1) und der zweite Dosierbehälter (100-2) voneinander trennbar sind.

15. Verfahren zum Dosieren von viskosen Materialkomponenten (103-1, 103-2), mit den Schritten:
Drücken (S101) der Materialkomponente (103-1, 103-2) aus einem Vorratsbehälter (111-1, 111-2) in eine Dosierkammer (107-1, 107-2); und
Ausgeben (S102) der Materialkomponente (103-1, 103-2) aus der Dosierkammer (107-1, 107-2) über einen Dosierstößel (109-1, 109-2).
